# NEW EUROPEAN PATENT SPECIFICATION

(11) **EP 1 632 216 B2**
(45) Date of publication and mention of the opposition decision: **18.09.2013**
(45) Mention of the grant of the patent: 14.07.2010
(21) Application number: 05017209.7
(22) Date of filing: 08.08.2005
(51) Int. Cl.: A61K 8/36, A61K 8/34, A61Q 15/00, A61K 8/04

(54) **Deodorant or antiperspirant sprays containing unsaturated fatty acids**
Deodorant- bzw. Antiperspirant-Sprays enthaltend ungesättigte Fettsäuren
Compositions aérosol désodorantes ou antiperspirantes comprenant des acides gras insaturés

(30) Priority: 09.08.2004 JP 2004232350
(43) Date of publication of application: 08.03.2006
(73) Proprietor: KAO CORPORATION, Chuo-ku, Tokyo (JP)
(72) Inventor: Fuji, Akira, 2-1-3, Bunka Sumida-ku Tokyo (JP); Hagura, Toyoki, 2-1-3, Bunka Sumida-ku Tokyo (JP)
(74) Representative: HOFFMANN EITLE

(56) References cited:
- EP-A- 0 550 960
- EP-A- 1 430 880
- EP-A- 1 520 576
- EP-A- 1 547 576
- WO-A-98/33476
- WO-A-02/102337
- GB-A- 1 282 889
- GB-A- 1 582 731
- PATENT ABSTRACTS OF JAPAN vol. 012, no. 143 (C-492), 30 April 1988 (1988-04-30) -& JP 62 257982 A (TOSHIHIRO IJICHI), 10 November 1987 (1987-11-10)

## Description

### BACKGROUND OF THE INVENTION

### 1. Field of the Invention

The present invention relates to deodorant spray preparations.

### 2. Description of the Prior Art

Deodorant products which are designed to prevent malodor and a sticky or tacky skin feeling each caused by perspiration have been widely used in the form of powder spray. Powder spray preparations are required not to form white residue caused by aggregation of powders sprayed onto the skin and not to provide unpleasant skin feeling such as sticky or tacky feeling. To meet such requirements, several techniques have been hitherto proposed.

For example, a liquid antiperspirant composition containing an antiperspirant such as aluminum hydroxychloride, an alcohol and a polyether-modified silicone is described in JP 5-229925A. The proposed antiperspirant composition is designed to prevent the formation of white residues of antiperspirant particles by the incorporation of the polyether-modified silicone and the alcohol. However, when a powdery material, in addition to the antiperspirant, is further incorporated to enhance a skin feeling such as silky feeling, the proposed antiperspirant composition fails to exhibit a sufficient effect on the prevention of white resides.

A powder aerosol composition containing an inorganic powder such as talc, silicic anhydride, kaolin, and aluminum hydroxychloride combined with a nonionic surfactant or a silicone oil is described in JP 3-157327A. Although effective for preventing the formation of white residues upon spraying, the proposed powder aerosol composition provides a strong oily feeling and fails to provide a silky feeling attributable to the powdery material.

In addition, an antiperspirant/deodorant composition incorporated with powders of spherical silicone rubber is described in JP 8-12545A and US 5628989, and an aerosol composition containing composite powders made of different kinds of organopolysiloxanes is described in JP 11-335254A.

GB-A-1 582 731 describes anti-odorant compositions comprising a substituted-phenolic antioxidant and a hydroxy-carboxylic acid compound. GB-A-1 282 889 relates to deodorant compositions comprising the salt of an unsaturated aliphatic hydroxycarboxylic acid. EP 0 550 960 is directed to antiperspirant materials and compositions that form, upon contact with perspiration, a water-insoluble liquid crystal phase. WO-A-02 102 337 discloses antiperspirant or deodorant compositions comprising an antiperspirant active comprising an astringent aluminium or zirconium salt, as well as a carrier for said antiperspirant active and a PPAR activating fatty acid or a hydrolysable precursor of the PPAR activating fatty acid. JP-A-62 257 982 is directed to deodorant antioxidant compositions comprising an oxicarboxylic acid, an (un)saturated fatty acid, L-ascorbic acid, erythorbic acid and their salt that can be used in fumy or liquid form. EP-A-1 520 576 reveals deodorant compositions comprising a plant extract and an unsaturated fatty acid. EP-A-1 547 576 is about antiperspirant and deodorant stick compositions containing an antiperspirant active, a structurant and a selected amount of an unsaturated fatty acid.

Although these compositions provide a silky feeling on its use, the prevention of white residues is insufficient.

### SUMMARY OF THE INVENTION

The inventors have found that a deodorant spray preparation containing an antiperspirant and/or an antimicrobial agent which is further incorporated with an unsaturated fatty acid and an antioxidant prevents the formation of white residues upon spraying while providing a silky skin feel with little tackiness and stickiness.

Thus, the present invention provides a deodorant spray preparation containing (A) an antiperspirant and/or an antimicrobial agent, (B) an unsaturated fatty acid, (C) an antioxidant, and (D) a propellant
wherein the component (B) is at least one fatty acid selected from myristoleic acid, palmitoleic acid, oleic acid, linoleic acid and linolenic acid,
wherein the content of the component (B) is from 0.01 to 1 % by weight based on the total amount of the deodorant spray preparation including the propellant (D),
wherein the content of the component (C) is from 0.0001 to 0.1 % by weight based on the total amount of the deodorant spray preparation including the propellant (D), and
wherein the content ratio of the component (C) and the component (B), (C)/(B), is from 1/100 to 1/2
as specified in claim 1.

### DETAILED DESCRIPTION OF THE INVENTION

The deodorant spray preparation of the present invention (hereinafter also referred to as "composition of the present invention") contains an antiperspirant and/or an antimicrobial agent as the component (A). Examples of the antiperspirants include aluminum chlorohyclrate, aluminum-zirconium composite salts, and aluminum chloride. Examples of the antimicrobial agents include triclosan, isopropylmethylphenol, benzoic acid, benzalkonium chloride, and trichlorocarbanilide. Each of the antiperspirants and the antimicrobial agents may be used alone or in combination of two or more. The antiperspirant and the antimicrobial agent may be used in combination.

The content of the antiperspirant is preferably from 0.01 to 5% by weight and more preferably from 0.05 to 3% by weight, and the content of the antimicrobial agent is preferably from 0.005 to 0.2% by weight and more preferably from 0.01 to 0.1% by weight, each based on the total amount of the composition including the propellant (D).

An unsaturated fatty acid is used as the component (B) of the composition of the present invention, wherein the component (B) is at least one fatty acid selected from myristoleic acid, palmitoleic acid, oleic acid, linoleic acid and linolenic acid.

The composition of the present invention contains one or more kinds of such unsaturated fatty acids. The content of the unsaturated fatty acid is from 0.01 to 1% by weight, preferably from 0.02 to 0.5% by weight, and more preferably from 0.05 to 0.3% by weight, each based on the total amount of the composition including the propellant (D). If the content is 0.01% by weight or more, the composition exerts the effect of preventing the formation of white residues. If the content is 1% by weight or less, a pleasant skin feeling with less oiliness is provided without generating specific fatty acid odor.

The composition of the present invention contains an antioxidant as the component (C) to enhance the long-lasting deodorant effect. Examples of the antioxidants include dibutylhydroxytoluene (BHT), butylhydroxyanisole, vitamin E and its derivatives, catechins, flavonoids, and polyphenols. These antioxidants may be used alone or in combination of two or mere.

In view of the balance between long-lasting deodorant effect and production economy, etc., the content of the antioxidant is from 0.0001 to 0.1% by weight and more preferably from 0.0005 to 0.03% by weight, each based on the total amount of the composition including the propellant (D).

The ratio of the contents of the component (C) and the component (B), (C)/(B), is from 1/100 to 1/2 and more preferably from 1/50 to 1/5. If the ratio (C)/(B) is 1/100 or more, the long-lasting deodorant effect is enhanced sufficiently, while a ratio of 1/2 or less is preferred in view of production economy.

In the present invention, the formation of white residues of powdery components such as the component (A) is effectively prevented by formulating a composition containing the components (A), (B) and (C) into the deodorant spray preparation.

For formulating the deodorant spray preparation, a propellant is used as the component (D). Examples of the propellants include freon gases such as freon 134a and freon 113; hydrocarbon gases such as propane, isobutane and isopentane; liquefied gases such as dimethyl ether; carbon dioxide gas; and nitrogen gas. The content of the propellant (D) is preferably from about 60 to about 99% by weight based on the total amount of the composition of the present invention.

The composition of the present invention optionally contains an oil that is in liquid state at room temperature, as long as the effects of the present invention are not adversely affected. Examples of such oils include silicone oils such as dimethylsilicone, decamethylcyclopentasiloxane, methylphenylsilicone and methylcyclopolysiloxane; modified silicone oils such as polyoxyethylene-methylpolysiloxane copolymers; and fatty acid esters such as myristyl myristate, isopropyl myristate, isopropyl palmitate and neopentyl glycol dicaprylate.

These oils may be used alone or in combination of two or more. The content of the oil, if used, is preferably from 0.1 to 25% by weight and more preferably from 0.3 to 15% by weight, each based on the total amount of the composition including propellant.

The composition of the present invention may contain, if necessary, an inorganic or organic powder other than the component (A). Examples of the inorganic powders include talc, sericite, mica, kaolin, red iron oxide, clay, bentonite, silicic anhydride, and synthetic silica beads. Examples of the organic powders include silicone resins such as vinyl dimethicone/methicone silsesquioxane crosspolymers (block copolymers of crosslinked silicone and reticular silicone) and methicone silsesquioxane (methylsiloxane reticular polymers), for example, "KMP-590" manufactured by Shin-Etsu Chemical Co., Ltd., "Tospearl® 145" and "Tospearl® 2000B" each manufactured by GE Toshiba Silicones, and "Trefil®" manufactured by Dow Corning Toray Silicones Co., Ltd.); nylon resins (for example, "SP-500" manufactured by Toray Industries Inc.); polystyrene resins (for example, "Finepearl" manufactured by Sumitomo Chemical Co., Ltd., "Techpolymer SB" manufactured by Sekisui Plastics Co., Ltd. and "Fine Powder SGP" manufactured by Soken Chemical & Engineering Co., Ltd.); polyethylene resins (for example, "Flo-Beads®" manufactured by Sumitomo Seika Chemicals Co., Ltd.); polymethyl methacrylate resins (for example, "Matsumoto Microsphere M" manufactured by Matsumoto Yushi-Seiyaku Co., Ltd., "Techpolymer MB" manufactured by Sekisui Plastics Co., Ltd. and "Fine Powder MP" manufactured by Soken Chemical & Engineering Co., Ltd.); divinylbenzene resins; polyurethane resins; benzoguanamine resins; melamine resins; phenol resins; fluorine-containing resins and fine polymer particles prepared by the dispersion polymerization of vinyl monomer in a solvent in the presence of a dispersing agent such as a polysiloxane compound that is monoterminated with a radical-polymerizable group (JP 11-181003A).

These powders may be used alone or in combination of two or more. The content of the powder, if used, is preferably from 0.1 to 25% by weight and more preferably from 0.3 to 15% by weight, each based on the total amount of the composition including propellant (D).

The composition of the present invention may further contain, if necessary, polyhydric alcohol such as glycerol, 1,3-butylene glycol and 1,4-butylene glycol, pigment, dyestuff, fragrance, cooling agent, anti-inflammatory agent, humectant, stabilizer, etc., as long as the use thereof is not adversely affect the effects of the present invention.

The following examples further describe and demonstrate embodiments of the present invention.

### EXAMPLES 1-6 and COMPARATIVE EXAMPLES 1-3

Each deodorant spray preparation having the formulation shown in Table 1 was prepared in a manner described below. The amounts given in Table 1 are expressed as percentages by weight.

The components except for the component (D) propellant shown in Table 1 were mixed uniformly. The resultant uniform mixture was poured into a spray container, which was then filled with LPG to prepare each deodorant spray preparation.

### Evaluation Method

A given amount of the deodorant spray preparation was uniformly applied by spraying to the upper arms of ten expert panelists. Each panelist organoleptically evaluated the retention of silky feeling and the formation of white residues according to the following scores. The scores reported by the panelists were summed up and the results were ranked according to the averaged scores. The evaluation results arc shown in Table 1 together with the formulations of the deodorant spray preparations.
(1) Retention of silky feeling (evaluated by the degree of silky feeling after one hour of application)
   Evaluation Scores
   5: silky
   4: somewhat silky
   3: difficult to determine whether silky or not
   2: not so silky
   1: not silky
   Ranks based on Averaged Scores
   ⊚: 4.5 to 5.0
   ○: 3.5 to 4.4
   ○Δ: 2.5 to 3.4.
   Δ: 1.5 to 2.4
   ×: 1.0 to 1.4
(2) Formation of White Residues (visually evaluated by the degree of whiteness immediately after application)
   Evaluation Scores
   4: not white
   3: not so white
   2: somewhat white
   1: white
   Ranks based on Averaged Scores
   ⊚: 3.5 to 4.0
   ○: 2.5 to 3.4
   Δ: 1.5 to 2.4
   ×: 1.0 to 1.4

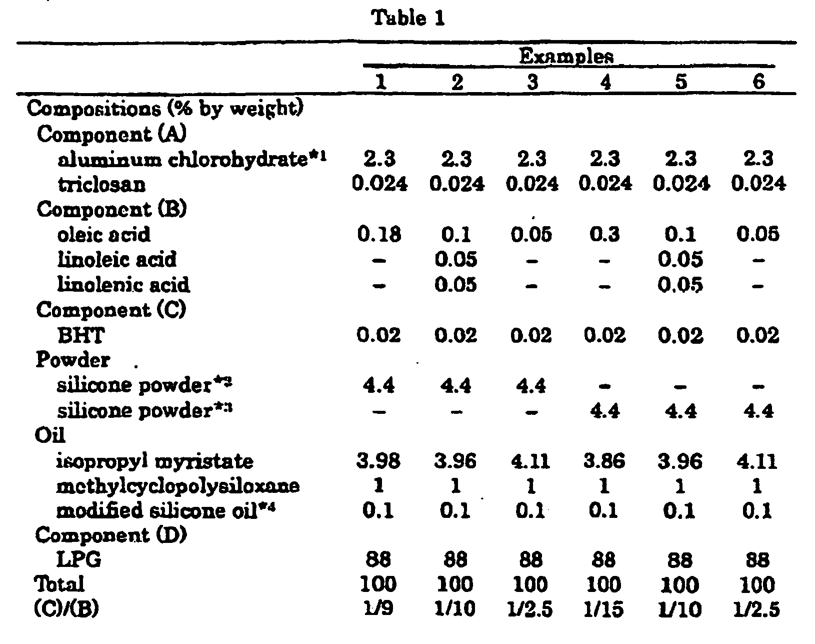

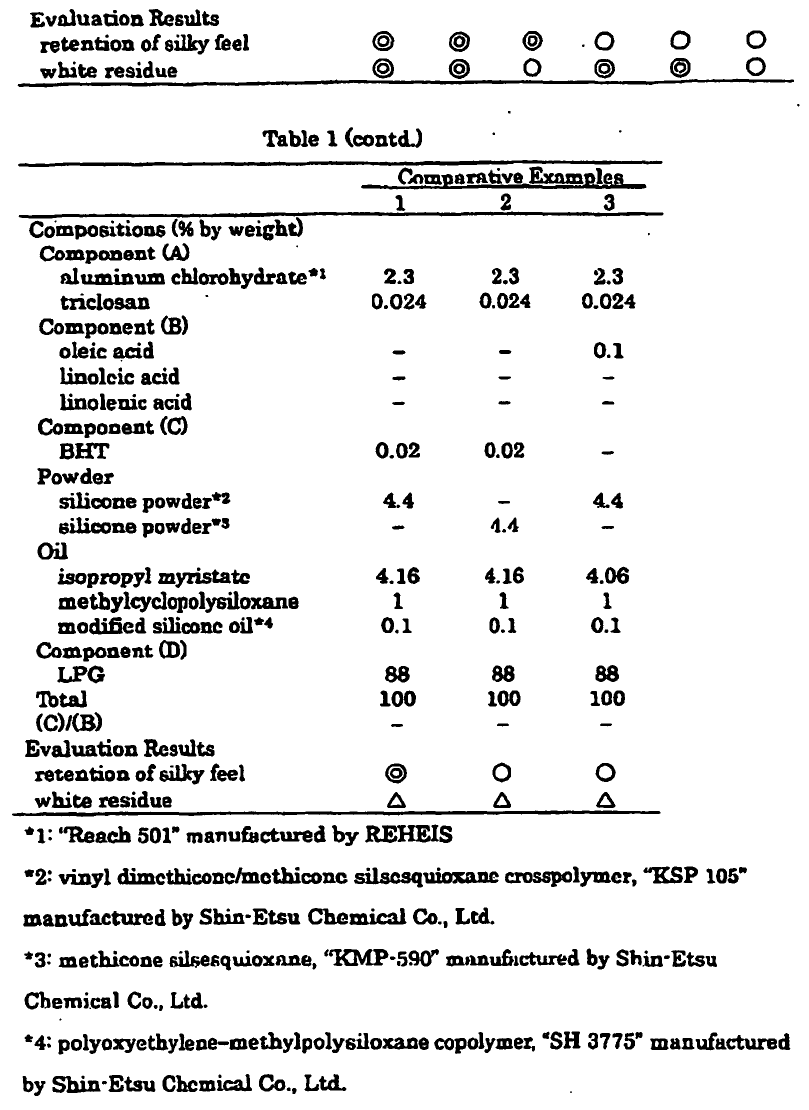

The results show that the deodorant spray preparations of the present invention, which contain an antiperspirant and/or an antimicrobial agent, an unsaturated fatty acid, an antioxidant, and a propellant, are excellent in both the retention of silky feeling and the effect for preventing the formation of white residues.

### INDUSTRIAL APPLICABILITY

The deodorant spray preparations of the present invention suppress the perspiration, prevent the formation of white residues due to aggregations of powders upon spraying, and provide a pleasant silky feeling with no unpleasant skin feeling such as tacky and sticky feeling.

## Claims

1. A deodorant spray preparation comprising (A) an antiperspirant and/or an antimicrobial agent, (B) an unsaturated fatty acid, (C) an antioxidant, and (D) a propellant,
wherein the component (B) is at least one fatty acid selected from myristoleic acid, palmitoleic acid, oleic acid, linoleic acid and linolenic acid,
wherein the content of the component (B) is from 0.01 to 1 % by weight based on the total amount of the deodorant spray preparation including the propellant (D) ,
wherein the content of the component (C) is from 0.0001 to 0.1 % by weight based on the total amount of the deodorant spray preparation including the propellant (D) , and
wherein the content ratio of the component (C) and the component (B) , (C)/(B), is from 1/100 to 1/2.

2. The deodorant spray preparation according to claim 1, further comprising a powder other than the component (A).

3. Use of a composition comprising (A) an antiperspirant and/or an antimicrobial agent, (B) an unsaturated fatty acid, (C) an antioxidant, and (D) a propellant,
wherein the component (B) is at least one fatty acid selected from myristoleic acid, palmitoleic acid, oleic acid, linoleic acid and linolenic acid,
wherein the content of the component (B) is from 0.01 to 1 % by weight based on the total amount of the deodorant spray preparation including the propellant (D),
wherein the content of the component (C) is from 0.0001 to 0.1 % by weight based on the total amount of the deodorant spray preparation including the propellant (D), and
wherein the content ratio of the component (C) and the component (B), (C)/(B), is from 1/100 to 1/2, as a deodorant spray preparation.

## Patentansprüche

1. Deodorantsprayzubereitung, umfassend (A) ein schweisshemmendes und/oder antimikrobielles Mittel, (B) eine ungesättigte Fettsäure, (C) ein Antioxidans und (D) ein Treibmittel,
wobei die Komponente (B) mindestens eine Fettsäure, ausgewählt aus Myristoleinsäure, Palmitoleinsäure, Ölsäure, Linolsäure und Linolensäure, ist,
wobei der Gehalt an Komponente (B) 0,01 bis 1 Gew.%, bezogen auf die Gesamtmenge der Deodorantsprayzubereitung, einschliesslich des Treibmittels (D), beträgt, und
wobei der Gehalt an Komponente (C) 0,0001 bis 0,1 Gew.%, bezogen auf die Gesamtmenge der Deodorantsprayzubereitung, einschliesslich des Treibmittels (D), beträgt, und
wobei das Anteilsverhältnis von Komponente (C) und Komponente (B), (C)/(B), 1:100 bis 1:2 beträgt.

2. Deodorantsprayzubereitung gemäss Anspruch 1, die ferner ein Pulver umfasst, das ein anderes als Komponente (A) ist.

3. Verwendung einer Zusammensetzung, umfassend (A) ein schweisshemmendes und/oder antimikrobielles Mittel, (B) eine ungesättigte Fettsäure, (C) ein Antioxidans und (D) ein Treibmittel,
wobei die Komponente (B) mindestens eine Fettsäure, ausgewählt aus Myristoleinsäure, Palmitoleinsäure, Ölsäure, Linolsäure und Linolensäure, ist,
wobei der Gehalt an Komponente (B) 0,01 bis 1 Gew.%, bezogen auf die Gesamtmenge der Deodorantsprayzubereitung, einschliesslich des Treibmittels (D), beträgt, und
wobei der Gehalt an Komponente (C) 0,0001 bis 0,1 Gew.%, bezogen auf die Gesamtmenge der Deodorantsprayzubereitung, einschliesslich des Treibmittels (D), beträgt, und
wobei das Anteilsverhältnis von Komponente (C) und Komponente (B), (C)/(B), 1:100 bis 1:2 beträgt,
als Deodorantsprayzubereitung.

## Revendications

1. Préparation aérosol déodorante comprenant (A) un anti-transpirant et/ou un agent antimicrobien, (B) un acide gras insaturé, (C) un antioxydant, et (D) un propulseur,
dans laquelle le composant (B) est au moins un acide gras choisi parmi l'acide myristoléique, l'acide palmitoléique, l'acide oléique, l'acide linoléique et l'acide linolénique,
dans laquelle la teneur du composant (B) est de 0,01 à 1% en poids sur la base de la quantité totale de la préparation aérosol déodorante incluant le propulseur (D),
dans laquelle la teneur du composant (C) est de 0,0001 à 0,1% en poids sur la base de la quantité totale de la préparation aérosol déodorante incluant le propulseur (D), et
dans laquelle le rapport des teneurs du composant (C) et du composant (B), (C)/(B), est de 1/100 à ½.

2. Préparation aérosol déodorante selon la revendication 1, comprenant en outre une poudre autre que le composant (A)

3. Utilisation d'une composition comprenant (A) un anti-transpirant et/ou un agent antimicrobien, (B) un acide gras insaturé, (C) un antioxydant, et (D) un propulseur,
dans laquelle le composant (B) est au moins un acide gras choisi parmi l'acide myristoléique, l'acide palmitoléique, l'acide oléique, l'acide linoléique et l'acide linolénique,
dans laquelle la teneur du composant (B) est de 0,01 à 1% en poids sur la base de la quantité totale de la préparation aérosol déodorante incluant le propulseur (D),
dans laquelle la teneur du composant (C) est de 0,0001 à 0,1% en poids sur la base de la quantité totale de la préparation aérosol déodorante incluant le propulseur (D), et
dans laquelle le rapport des teneurs du composant (C) et du composant (B), (C)/(B), est de 1/100 à ½
comme une préparation aérosol déodorante.
